# EUROPEAN PATENT APPLICATION

(11) **EP 4 062 887 A1**
(43) Date of publication of application: **28.09.2022**
(21) Application number: 20907834.4
(22) Date of filing: 30.11.2020
(51) Int. Cl.: A61H 1/02, A61B 5/11, A61M 21/00

(54) **MIRROR TREATMENT DEVICE AND MIRROR TREATMENT SYSTEM**

(30) Priority: 26.12.2019 CN 201911370483
(71) Applicant: Shentai Medical Technology (Shanghai) Co., Ltd., Shanghai 201203 (CN)
(72) Inventor: TIAN, Tian, Shanghai 201203 (CN); GAO, Zhijun, Shanghai 201203 (CN); WANG, Hongmei, Shanghai 201203 (CN); ZHOU, Tong, Shanghai 201203 (CN)
(74) Representative: Marks & Clerk LLP
(86) International application number: PCT/CN2020/132949
(87) International publication number: WO 2021/129321

(57) **Abstract**

A mirror therapy device includes a first positioning assembly, a second positioning assembly and a transmission assembly connecting the first and second positioning assemblies. The first and second positioning assemblies are arranged in opposition to each other in such a manner that both are able to reciprocate. The first positioning assembly is configured for positioning of a healthy limb of a patient, and the second positioning assembly is configured for positioning of an affected limb of the patient. The transmission assembly is configured for power transmission between the first and second positioning assemblies so that the second positioning assembly moves in synchronization with the first positioning assembly under the action of the transmission assembly. A mirror therapy system includes the mirror therapy device and a display device. The mirror therapy system enables the healthy limb to drive movement of the affected limb to allow the affected limb to repeat the same movements as the healthy limb, thus establishing neural connections for synchronous vision, motor perception and proprioception of the patient's healthy and affected sides and better stimulating nerve recovery of the patient.

## Description

### TECHNICAL FIELD

The present invention relates to the technical field of medical devices and, in particular, to a mirror therapy device and a mirror therapy system.

### BACKGROUND

With the acceleration of our pace of life and the aging of the population, the incidence of stroke is increasing year by year, and 75% of poststroke patients remain with limb dysfunction. Apart from limb paralysis caused by brain damage itself, complications such as shoulder pain, shoulder-hand syndrome and spasticity can also affect limb functional recovery. For limb functional recovery of stroke patients, upper limb function is particularly important because it is closely related to the patients' activities of daily living (ADLs) and upper limb functional recovery is directly related to the patients' quality of life and return to the society.

Mirror therapy, also known as mirror visual feedback therapy, which was first proposed by Ramachandran and other scholars in 1995, is used in the practice of rehabilitation medicine and neuroscience research. It mainly consists of separating the healthy arm of a patient from the affected arm with a mirror so that when the patient is moving the healthy hand, he/she will imagine, based on the arm's image in the mirror, that the affected hand is actually also moving. This can promote the patient's nerve recovery.

With the development of robotics in recent years, robot and vision technologies are being gradually applied to mirror therapy devices. Through robot and vision technologies, motor perception and proprioception are introduced to enhance the effect of mirror therapy. That is, by means of electric motor drive and myoelectric signal detection, and through detecting healthy limb movements, the affected limb is driven to make the same movements to accomplish nerve recovery for the patient's vision, motor perception and proprioception. However, due to inaccuracy and latency in signal detection, the patient will perceive obvious asynchrony in movements of the affected limb, and it is impossible to achieve synchronous motor-perceptive and proprioceptive stimulation of the healthy and affected limbs as required for better promotion of the patient's nerve recovery.

### SUMMARY

It is an aim of the present invention to provide a mirror therapy device and mirror therapy system, which address the problem of availability of only visual stimulation and absence of motor-perceptive and proprioceptive stimulation associated with conventional mirror therapy approaches.

In pursuit of the above aim, the present invention provides a mirror therapy device including a first positioning assembly, a second positioning assembly and a transmission assembly connecting the first and second positioning assemblies,
the first and second positioning assemblies arranged in opposition to each other in such a manner that both are able to reciprocate, the first positioning assembly adapted for positioning of a healthy limb of a patient, the second positioning assembly adapted for positioning of an affected limb of the patient,
the transmission assembly adapted for power transmission between the first and second positioning assemblies so that the second positioning assembly moves in synchronization with the first positioning assembly.

Optionally, the first positioning assembly may include at least one first finger ring and the second positioning assembly may include at least one second finger ring each arranged in correspondence with a respective one of the at least one first finger ring, with the transmission assembly including at least one set of transmission elements each adapted to maintain a respective one of the at least one first finger ring and a respective one of the at least one second finger ring that is arranged in correspondence with the respective finger ring in synchronous movement.

Optionally, the transmission elements may include a transmission cord capable of reciprocating and tied together at both ends to form a loop, wherein the first finger ring is attached to a first cord segment of the transmission cord and the second finger ring is attached to a second cord segment of the transmission cord, and wherein the first cord segment to which the first finger ring is attached is made in the same movement state as the second cord segment to which the second finger ring is attached.

Optionally, the first cord segment to which the first finger ring may be oriented at a first angle with respect to a horizontal plane and the second cord segment to which the second finger ring may be oriented at a second angle with respect to the horizontal plane, both the first and second angles being greater than 0° and less than 90°.

Optionally, the first finger ring may include, connected to each other, a first ring body and a first connecting member and the second finger ring may include, connected to each other, a second ring body and a second connecting member, with the transmission cord including a first transmission cord body and a second transmission cord body, the first transmission cord body connected to the first ring body at one end and to the second connecting member at the other end, the second transmission cord body connected to the second ring body at one end and to the first connecting member at the other end.

Optionally, the transmission element may further include a plurality of first guide elements and a plurality of second guide elements, the plurality of first guide elements arranged in correspondence with the first finger ring, the plurality of second guide elements arranged in correspondence with the second finger ring, wherein the transmission cord is successively wound on the plurality of first guide elements and the plurality of second guide elements so as to form the loop and be able to reciprocate relative to the first and second guide elements.

Optionally, the first and second guide elements may all be pulleys.

Optionally, the mirror therapy device may further include at least one displacement sensor arranged in correspondence with, and adapted to measure displacement of, the transmission cord.

Optionally, the displacement sensor may be a photoelectric displacement sensor, wherein the transmission cord is provided thereon with a mark corresponding to the displacement sensor.

Optionally, the mirror therapy device may further include a first support pad for supporting the healthy limb and a second support pad for supporting the affected limb, the first support pad arranged in correspondence with the first positioning assembly, the second support pad arranged in correspondence with the second positioning assembly.

Optionally, the first support pad may be provided with a first concave surface on its side intended to be brought into contact with the healthy limb, and the second support pad may be provided with a second concave surface on its side intended to be brought into contact with the affected limb.

Optionally, the mirror therapy device may further include at least one first camera and at least one second camera, the first camera arranged in correspondence with the first positioning assembly, the second camera arranged in correspondence with the second positioning assembly.

Optionally, the mirror therapy device may further include a lighting lamp.

Optionally, the mirror therapy device may further include a box body defining an internal cavity where the first positioning assembly, the second positioning assembly and the transmission assembly are all disposed.

Optionally, the box body may include a support frame and, all mounted on the support frame, a first side plate, a second side plate, a top plate, a back panel and a bottom plate.

Optionally, a gap may be left between the bottom plate and the bottom of the support frame so that the bottom plate is raised above the bottom of the support frame.

Optionally, the box body further may include a front panel mounted on the support frame, the front panel arranged in opposition to the back panel, the front panel provided therein with a first aperture adapted for passage of the healthy limb therethrough and a second aperture adapted for passage of the affected limb therethrough, the first aperture arranged in correspondence with the first positioning assembly, the second aperture arranged in correspondence with the second positioning assembly.

In pursuit of the above aim, the present invention also provides a mirror therapy system including the mirror therapy device as described above and a display device, the display device adapted to display images of movements of the healthy and affected limbs.

The mirror therapy device and system provided in the present invention has the following advantages over the prior art:
1) The mirror therapy device provided in the present invention includes a first positioning assembly, a second positioning assembly and a transmission assembly connecting the first and second positioning assemblies, the first and second positioning assemblies arranged in opposition to each other in such a manner that both are able to reciprocate, the first positioning assembly adapted for positioning of a healthy limb of a patient, the second positioning assembly adapted for positioning of an affected limb of the patient, the first and second positioning assemblies relying on the transmission assembly for power transmission therebetween so that the second positioning assembly is able to move in synchronization with the first positioning assembly under the action of the transmission assembly. Therefore, with the mirror therapy device provided in the present invention, the healthy limb can drive the affected limb under the action of the transmission assembly to enable the affected limb to synchronously repeat the same movements as the healthy limb to establish neural connections for synchronous vision, motor perception and proprioception of the patient's healthy and affected sides and better stimulate nerve recovery of the patient, thus addressing the problem of availability of only visual stimulation and absence of motor-perceptive and proprioceptive stimulation associated with conventional mirror therapy approaches.
2) The mirror therapy device provided in the present invention employs a transmission cord as a transmission element in lieu of a motor transmission system. As a result, not only the mirror therapy device can be fabricated at significantly reduced cost, but it can also be ensured that the affected and healthy limbs move in synchronization, thus effectively avoiding asynchronous movements of the affected and healthy limbs due to inaccuracy and latency in signal detection.
3) The mirror therapy device provided in the present invention employs pulleys as guide elements for the transmission cord. This can not only effectively reduce friction of the transmission cord and hence discomfort of the patient during limb training but also further ensure synchrony of movement of the affected and healthy limbs.
4) The mirror therapy device provided in the present invention employs a photoelectric displacement sensor for movement and displacement measurement of the transmission cord. Compared with conventional inertial sensors, the photoelectric displacement sensor provides faster response and higher measurement accuracy, less suffers from zero shifting and is more reliable. It can measure limb movements in real time, providing a basis for interesting rehabilitation.
5) The mirror therapy device provided in the present invention employs a first camera capable of capturing movements of the healthy limb in real time and a second camera capable of capturing movements of the affected limb in real time. Moreover, it employs an image compensation algorithm for processing the captured images to remove the first positioning assembly, the second positioning assembly and the transmission assembly therefrom, thus avoiding the patient from knowing the transmission mechanism and enabling mirror therapy to provide an increased "sense of reality". Further, tactile differences perceived by the patient during transmission can be compensated for by adding immersion from interesting training, in order to further enhance the patient's nerve recovery.
6) Since the mirror therapy system provided in the present invention includes the mirror therapy device as described above, it has all the advantages of the mirror therapy device. The mirror therapy system provided by the present invention enables the healthy limb to drive the affected limb to allow the affected limb to repeat the same movements as the healthy limb, thus establishing neural connections for the patient's vision, motor perception and proprioception and better stimulating nerve recovery of the patient.

### BRIEF DESCRIPTION OF THE DRAWINGS

Fig. 1 is a schematic structural overview of the mirror therapy device according to an embodiment of the present invention;
Fig. 2 is a schematic diagram showing part of the structure of the mirror therapy device according to an embodiment of the present invention;
Fig. 3 is a schematic structural diagram showing connection relationships among a first positioning assembly, a second positioning assembly and a transmission assembly in the mirror therapy device according to an embodiment of the present invention; and
Fig. 4 is a schematic structural diagram showing a power-transmission relationship between one first finger ring and one second finger ring in the mirror therapy device according to an embodiment of the present invention.

The following is a list of reference numerals used in the drawings:
100-First Finger Ring; 200-Second Finger Ring; 310-Transmission Cord; 311-First Cord Segment; 312-Second Cord Segment; 110-First Ring Body; 120-First Connecting member; 210-Second Ring Body; 220-Second Connecting member; 313-First Transmission Cord Body; 314-Second Transmission Cord Body; 320-First Guide Element; 330-Second Guide Element; 400-Displacement Sensor; 510-First Support Pad; 520-Second Support Pad; 511-First Concave Surface; 521-Second Concave Surface; 610-First Camera; 620-Second Camera; 700-Box Body; 710-Support Frame; 720-First Side Plate; 730-Second Side Plate; 740-Top Plate; 750-Back Panel; 760-Bottom Plate; 770-Front Panel; 771-First Aperture; 772-Second Aperture; 340-Support Mount; 711-Support Element; 712-First Support Pillar; 713-Second Support Pillar; 761-First Cutout; 762-Second Cutout; 741-First Through Hole; 742-Second Through Hole; 810-First Glove; 820-Second Glove.

### DETAILED DESCRIPTION

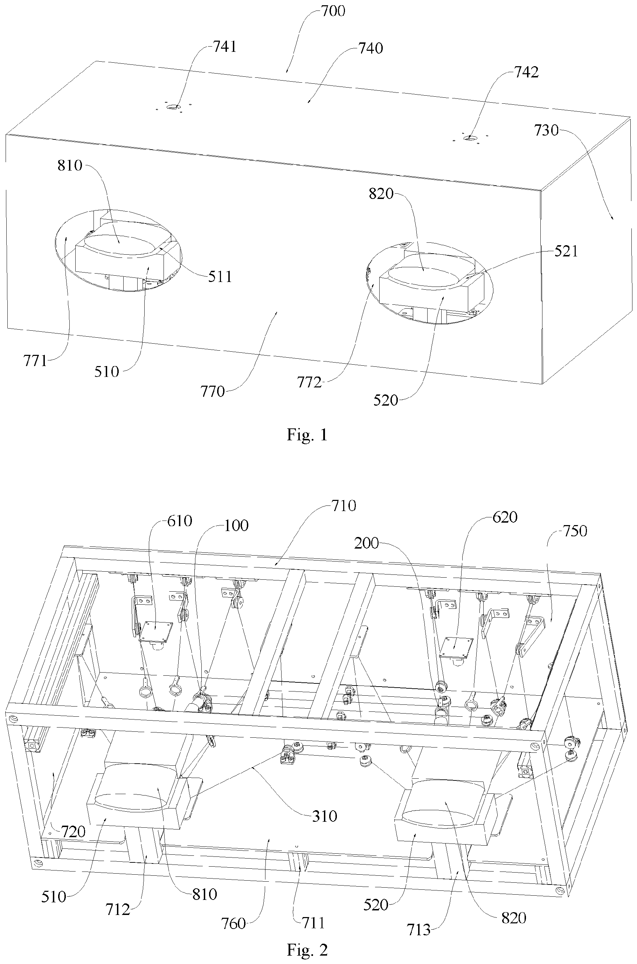

The mirror therapy device and system proposed in the present invention will be described in greater detail below with reference to Figs. 1 to 4 and specific embodiments. From the following description, advantages and features of the present invention will become more apparent. Note that the drawings are provided in a very simplified form not necessarily drawn to exact scale for the only purpose of helping to explain the disclosed embodiments in a more convenient and clearer way. In order that objects, features and advantages of the present invention may become readily apparent, reference is made to the accompanying drawings. It should be noted that architectural, proportional, dimensional and other details in the figures are presented only for the purpose of facilitating, in conjunction with the disclosure herein, the understanding and reading of those familiar with the art rather than being intended to limit conditions under which the present invention can be implemented. Therefore, they are technically of no substantive significance, and any and all architectural modifications, proportional variations or dimensional changes that do not affect the benefits and objects of the present invention are considered to fall within the scope of the teachings herein.

It is to be noted that, as used herein, relational terms such as first and second, etc., are only used to distinguish one entity or operation from another entity or operation, and do not necessarily require or imply these entities having such an order or sequence. Moreover, the terms "include," "including," or any other variations thereof are intended to cover a non-exclusive inclusion within a process, method, article, or apparatus that includes a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element.

In the description herein, it would be appreciated that the orientational or positional relationships described by the terms "central", "longitudinal", "transverse", "length", "width", "thickness", "upper", "lower", "front", "rear", "left", "right", "vertical", "horizontal", "top", "bottom", "inner", "outer", "axial", "radial", "circumferential", etc. are based on the orientations or positions shown in the accompanying drawings. They are intended merely to facilitate and simplify the explanation of the invention and do not indicate or imply that the stated components or elements have to assume, or be constructed or operated in, particular orientations. Therefore, they are not to be construed as limiting the invention. In the description herein, unless otherwise specified, the meaning of "plurality" is two or more.

In the description herein, unless otherwise expressly specified and defined, the terms "mounting", "coupling", "connection" and "securing" should be interpreted in a broad sense. For example, a connection may be a permanent, detachable or integral connection, or a mechanical or electrical connection, or a direct or indirect connection with one or more intervening media, or an internal communication or interaction between two components. Those of ordinary skill in the art can understand the specific meanings of the above-mentioned terms herein, depending on their context.

In the description herein, unless otherwise expressly specified and defined, when a first feature is described as being "on" or "under" a second feature, it can be in direct contact with the second feature, or intervening feature(s) may also be present. Moreover, when a first feature is described as being "over", "overlying" or "above" a second feature, it may either be situated normally or obliquely over the second feature, or it may only be located at a horizontal height higher than a horizontal height at which the second feature is located. When a first feature is described as being "under", "underlying" or "beneath" a second feature, it may either be situated normally or obliquely under the second feature, or it may only be located at a horizontal height lower than a horizontal height at which the second feature is located.

The core concept of the present invention is to provide a mirror therapy device and a mirror therapy system, which address the problem of availability of only visual stimulation and absence of motor-perceptive and proprioceptive stimulation associated with conventional mirror therapy approaches.

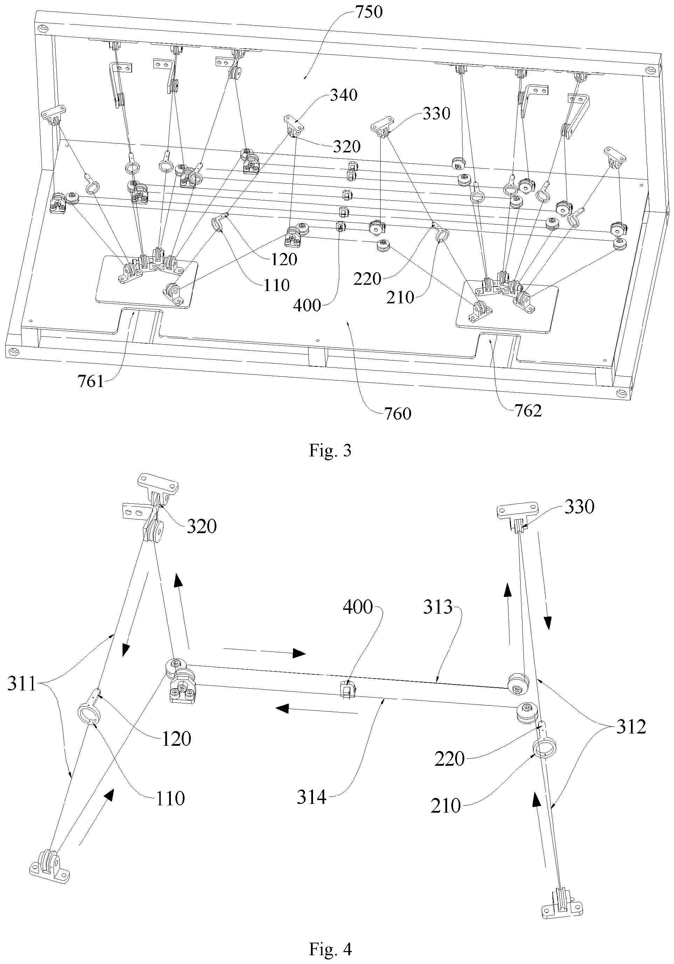

According to the above concept, the present invention provides a mirror therapy device. Reference is now made to Fig. 1, a schematic structural overview of the mirror therapy device according to an embodiment of the present invention, Fig. 2, a schematic diagram showing part of the structure of the mirror therapy device according to an embodiment of the present invention, and Fig. 3, a schematic structural diagram showing connection relationships among a first positioning assembly, a second positioning assembly and a transmission assembly in the mirror therapy device according to an embodiment of the present invention. As shown in Figs. 1 to 3, the mirror therapy device includes the first positioning assembly (not labeled), the second positioning assembly (not labeled) and the transmission assembly (not labeled) that connects the first positioning assembly to the second positioning assembly.

The first and second positioning assemblies are arranged in opposition to each other so that both can reciprocate. The first positioning assembly is adapted for positioning of a healthy limb of a patient, and the second positioning assembly is adapted for positioning of an affected limb of the patient. Power is transmitted between the first and second positioning assemblies by the transmission assembly. Under the action of the transmission assembly, the second positioning assembly is able to move in synchronization with the first positioning assembly. During a rehabilitation therapy, the patient first puts the healthy limb and the affected limb on the first and second positioning assemblies, respectively, and then makes movements of the healthy limb. Under the action of the transmission assembly, the second positioning assembly can move in synchronization with the first positioning assembly, enabling the affected limb to repeat the same movements as the healthy limb. Taking hand rehabilitation as an example, during a hand rehabilitation therapy, the patient first puts the healthy and affected hands on the first and second positioning assemblies, respectively, and then makes a flexion movement of the healthy hand, which cause a movement of the first positioning assembly. Under the action of the transmission assembly, the second positioning assembly moves in synchronization with the first positioning assembly, enabling the affected hand to make a synchronous flexion movement. Likewise, when the healthy hand is making an extension movement, under the action of the transmission assembly, the affected hand will make a synchronous extension movement. It is to be noted that, although the description herein is made in the context of the hand being taken as an example of an object of rehabilitation, as would be appreciated by those skilled in the art, the object of rehabilitation may also be another limb than the hand, and the present invention is not limited thereto.

Thus, using the mirror therapy device provided in the present invention, the healthy limb can drive the affected limb under the action of the transmission assembly, enabling the affected limb to synchronously repeat the same movements as the healthy limb. This can establish neural connections for synchronous vision, motor perception and proprioception of the patient's healthy and affected sides and better stimulate nerve recovery of the patient, addressing the problem of availability of only visual stimulation and absence of motor-perceptive and proprioceptive stimulation associated with conventional mirror therapy approaches.

Preferably, as shown in Figs. 1 and 2, in order to facilitate manipulation, the mirror therapy device further includes a box body 700 defining an internal cavity, in which all the first positioning assembly, the second positioning assembly and the transmission assembly are arranged. Arranging the first positioning assembly, the second positioning assembly and the transmission assembly in the cavity of the box body 700 allows the mirror therapy device to be more conveniently manipulated and carried. In addition, mounting the first positioning assembly, the second positioning assembly and the transmission assembly in the cavity of the box body 700 makes it easier to conceal the transmission assembly and avoids the patient from knowing the transmission mechanism of the rehabilitation device, enabling mirror therapy to provide an increased "sense of reality", additionally promoting nerve recovery of the patient's motor perception and proprioception. For ease of description, in this embodiment, in Figs. 1 and 2, the first positioning assembly is arranged on the left and the second positioning assembly on the right. In other embodiments, they may also be arranged reversely, i.e., the second positioning assembly is arranged on the left and the first positioning assembly on the right.

Preferably, as shown in Figs. 1 and 2, the box body 700 includes a support frame 710 and, all mounted on the support frame 710, a first side plate 720, a second side plate 730, a top plate 740, a back panel 750 and a bottom plate 760. Configuring the box body 700 as a structure including the support frame 710 allows quick assembly of the box body 700 by mounting the first side plate 720, the second side plate 730, the top plate 740, the back panel 750 and the bottom plate 760 from the left, right, top, back and bottom sides of the support frame 710, respectively. Moreover, such a structure allows easier mounting of the first positioning assembly, the second positioning assembly and the transmission assembly.

Preferably, in order to facilitate assembly and disassembly, the first side plate 720, the second side plate 730, the top plate 740, the back panel 750 and the bottom plate 760 may be mounted on the support frame 710 using fasteners. More preferably, the first side plate 720, the second side plate 730, the top plate 740, the back panel 750 and the bottom plate 760 may be mounted on the support frame 710 using threaded fasteners.

Preferably, as shown in Fig. 2, a gap is left between the bottom plate 760 and the bottom of the support frame 710 so that the bottom plate 760 is raised above the bottom of the support frame 710. Specifically, a plurality of support elements 711 may be disposed on the bottom of the support frame 710, and the bottom plate 760 may be laid on the support elements 711 and thus raised above the bottom of the support frame 710. Since the first and second positioning assemblies are mounted in the cavity that is defined by the first side plate 720, the second side plate 730, the top plate 740, the back panel 750 and the bottom plate 760, raising the bottom plate 760 above the bottom of the support frame 710 elevates the entire cavity and positions the first and second positioning assemblies overall at a higher height, thus further facilitating the placement of the patient's healthy and affected limbs and further increasing convenience of manipulation. Further, the space between the bottom plate 760 and the bottom of the support frame 710 may be utilized to stow some components of the first positioning assembly, the second positioning assembly and the transmission assembly.

Preferably, as shown in Fig. 1, the box body 700 further includes a front panel 770 mounted on the support frame 710, the front panel 770 is in opposition to the back panel 750. The front panel 770 is provided with a first aperture 771 adapted for passage of the healthy limb therethrough and a second aperture 772 adapted for passage of the affected limb therethrough. The first aperture 771 is provided in correspondence with the first positioning assembly, and the second aperture 772 is provided in correspondence with the second positioning assembly. The front panel 770 further included in the box body 700 can provide concealment itself, dispensing with the use of any additional concealing component for concealing the transmission assembly during a rehabilitation therapy. This additionally enhances the therapeutic effectiveness and convenience of use of the mirror therapy device provided in the present invention. Moreover, providing the first aperture 771 for passage of the healthy limb therethrough and the second aperture 772 for passage of the affected limb therethrough in the front panel 770 allows the patient to, during a rehabilitation therapy, put the healthy limb into the box body 700 through the first aperture 771 and put the affected limb into the box body 700 through the second aperture 772. This results in an additional increase in convenience of manipulation. It is to be noted that, as would be appreciated by those skilled in the art, the box body 700 may be alternatively structured not to include the support frame 710. That is, the box body 700 is made up of only the first side plate 720, the second side plate 730, the front panel 770, the back panel 750, the top plate 740 and the bottom plate 760.

Preferably, as shown in Figs. 1 and 2, the mirror therapy device further includes a first support pad 510 for supporting the healthy limb and a second support pad 520 for supporting the affected limb. The first support pad 510 is provided in correspondence with the first positioning assembly, and the second support pad 520 is provided in correspondence with the second positioning assembly. Providing the first support pad 510 and the second support pad 520 allow the patient to, during a rehabilitation therapy, rest at least part of the healthy limb on the first support pad 510 and at least part of the affected limb on the second support pad 520. The support provided by the first support pad 510 and the second support pad 520 to the healthy limb and the affected limb, respectively, effectively reduces the patient's fatigue during the rehabilitation therapy, additionally improving the performance of the rehabilitation therapy. Continuing the example of hand rehabilitation, during the rehabilitation therapy, the patient may rest the wrist of the healthy hand on the first support pad 510 and the wrist of the affected hand on the second support pad 520. Support provided by the first support pad 510 and the second support pad 520 to the wrists of the healthy and affected hands, respectively, can help the patient better comply with the hand rehabilitation therapy.

Preferably, as shown in Fig. 2, in order to further facilitate manipulation and provide the patient with more comfort during rehabilitation training, both the first support pad 510 and the second support pad 520 are spaced from the bottom plate 760 of the box body 700 by a gap. That is, both the first support pad 510 and the second support pad 520 are raised above the bottom plate 760. In order to facilitate mounting of the first support pad 510 and the second support pad 520, the support frame 710 is provided with a first support pillar 712 adapted for fixation of the first support pad 510 and a second support pillar 713 adapted for fixation of the second support pad 520. The first support pad 510 may be mounted on the first support pillar 712 by fasteners, and the second support pad 520 may be mounted on the second support pillar 713 by fasteners. Preferably, the fasteners are threaded fasteners. In order to facilitate the arrangement of the first support pillar 712 and the second support pillar 713, in the bottom plate 760, a first cutout 761 engageable with the first support pillar 712 and a second cutout 762 engageable with the second support pillar 713 are provided.

Preferably, as shown in Figs. 1 and 2, a first concave surface 511 is provided on the side of the first support pad 510 where it is adapted to brought into contact with the healthy limb, and a second concave surface 521 is provided on the side of the second support pad 520 where it is adapted to brought into contact with the affected limb. The first concave surface 511 provided on the first support pad 510 and the second concave surface 521 provided on the second support pad 520 can not only better support the healthy and affected limbs but also allow desirable positioning of them. As a result, the patient can more easily place the healthy limb on the first positioning assembly and the affected limb on the second positioning assembly.

Preferably, as shown in Fig. 2, the mirror therapy device further includes at least one first camera 610 and at least one second camera 620. The first camera 610 is provided in correspondence with the first positioning assembly, and the second camera 620 is provided in correspondence with the second positioning assembly. The first camera 610 can automatically capture movements of the healthy limb in real time, and the second camera 620 can automatically capture movements of the affected limb in real time. Thus, not only real-time display of limb movements but also greater convenience of manipulation can be achieved. Images captured by the cameras can be processed using an image compensation algorithm to remove the first positioning assembly, the second positioning assembly and the transmission assembly therefrom to avoid the patient from knowing the transmission mechanism of the rehabilitation device, enabling mirror therapy to provide an increased sense of reality and additionally promoting nerve recovery of the patient's motor perception and proprioception. Preferably, in order to for higher imaging quality to be achieved, the first camera 610 and the second camera 620 are both mounted on the top plate 740 of the box body 700. It is to be noted that, as would be appreciated by those skilled in the art, the first camera 610 and the second camera 620 may also be mounted on the back panel 750, the front panel 770, the first side plate 720, the second side plate 730 or the bottom plate 760 of the box body 700, as actually needed, and the present invention is not limited any particular mounting location of the first camera 610 or the second camera 620. In other embodiments, the mirror therapy device may include only one camera with a field of view covering both the first and second positioning assemblies. This can additionally avoid the problem of asynchrony between images captured by a plurality of cameras.

Preferably, as shown in Fig. 1, in order to facilitate wiring, in the top plate 740 of the box body 700, a first through hole 741 in correspondence with the first camera 610 and a second through hole 742 in correspondence with the second camera 620 are provided.

Preferably, the mirror therapy device further includes a lighting lamp (not shown). The lighting lamp can provide supplemental lighting. In particular, when the box body 700 includes the front panel 770, the back panel 750, the top plate 740, the bottom plate 760, the first side plate 720 and the second side plate 730, the interior of the box body 700 will be rather dark. Arranging the lighting lamp can increase brightness inside the box body 700 and enables improved imaging quality of the first camera 610 and the second camera 620. More preferably, the lighting lamp is an LED lamp. Implementing the lighting lamp as an LED lamp can additionally increase brightness inside the box body 700 and enables additionally improved imaging quality of the first camera 610 and the second camera 620, making images captured by the first camera 610 and the second camera 620 more clearly visible.

Preferably, as shown in Figs. 2 and 3, the first positioning assembly includes at least one first finger ring 100, and the second positioning assembly includes at least one second finger ring 200. The first finger ring(s) 100 is/are arranged in correspondence with the respective second finger ring(s) 200. The transmission assembly includes at least one set of transmission elements. The correspondingly arranged first finger ring(s) 100 and respective second finger ring(s) 200 are maintained in synchronous movement by the respective set(s) of transmission elements. Since the first positioning assembly includes at least one first finger ring 100, the second positioning assembly includes at least one second finger ring 200 and each pair of correspondingly arranged first finger ring 100 and second finger ring 200 relies on a respective one of the set(s) of transmission elements for power transmission to maintain the correspondingly arranged first finger ring 100 and second finger ring 200 in synchronous movement, the mirror therapy device provided in the present invention can provide the patient with a better finger rehabilitation therapy. During the finger rehabilitation therapy, the patient may put the affected finger in need of the rehabilitation therapy in the second finger ring 200 so that the second finger ring 200 tightly fits over the affected finger and insert the healthy finger corresponding to the affected finger into the first finger ring 100 so that the first finger ring 100 tightly fits the healthy finger. Preferably, in order to improve efficacy of the rehabilitation therapy, the first sections of the healthy and affected fingers may be inserted in the first finger ring 100 and the second finger ring 200, respectively. When the healthy finger performs a flexion movement, the first finger ring 100 is pressed down, and under the action of transmission elements, the second finger ring 200 is synchronously pushed down, causing the affected finger to perform a synchronous flexion movement. When the healthy finger performs an extension movement, the first finger ring 100 is moved upward, and under the action of the transmission elements, the second finger ring 200 is synchronously moved upward, causing the affected finger to perform a synchronous extension movement.

Preferably, as shown in Figs. 2 and 3, in order to provide enhanced convenience of use and improved rehabilitation efficacy, the first positioning assembly includes five first finger rings 100 adapted to tightly fit respectively over the thumb, the index finger, the middle finger, the ring finger and the little finger of the patient's healthy hand, and the second positioning assembly includes five second finger rings 200 adapted to tightly fit respectively over the thumb, the index finger, the middle finger, the ring finger and the little finger of the patient's affected hand. The transmission assembly includes five sets of transmission elements adapted to transmit power respectively between the healthy and affected thumbs, between the healthy and affected index fingers, between the healthy and affected middle fingers, between the healthy and affected ring fingers and between the healthy and affected little fingers. This arrangement enables simultaneous treatment of the patient's five fingers, resulting in effectively improved rehabilitation efficiency and efficacy.

Preferably, as shown in Fig. 2, in order to facilitate wearing of the first finger rings 100 and the second finger rings 200, a first glove 810 (not fully shown in the figure) for deploying the first finger rings 100 and a second glove 820 (not fully shown in the figure) for deploying the second finger rings 200 may be arranged. More preferably, the five first finger rings 100 are disposed over first sections of respective five finger portions of the first glove 810, and the second finger rings 200 are disposed over first sections of respective five finger portions of the second glove 820. A wrist portion of the first glove 810 is placed on the first support pad 510, and a wrist portion of the second glove 820 is placed on the second support pad 520. In this way, during a rehabilitation therapy, the patient can manage to wear the first finger rings 100 over the fingers of the healthy hand and the second finger rings 200 over the fingers of the affected hand simply by inserting the healthy hand into the first glove 810 and affected hand into the second glove 820, providing more convenience of manipulation.

Preferably, reference is now made to Figs. 2 to 4. Fig. 4 is a schematic structural diagram showing a power-transmission relationship between one first finger ring 100 and one second finger ring 200 in the mirror therapy device according to an embodiment of the present invention, in which the arrows represent directions of movement of various cord segments in a transmission cord 310 during a flexion movement made by the healthy finger. As shown in Figs. 2 to 4, the transmission elements include the transmission cord 310, which can move forth and back and are tied together at both ends to form a loop. The first finger ring 100 is attached to a first cord segment 311 of the transmission cord 310, and the second finger ring 200 is attached to a second cord segment 312 of the transmission cord 310. The first cord segment 311 to which the first finger ring 100 is attached is in the same state of movement as the second cord segment 312 to which the second finger ring 200 is attached. Here, the state of movement includes direction, distance, time, start and finish, and the like of movement. Specifically, the first finger ring 100 and the second finger ring 200 may define two holes, in both of which the transmission cord 310 is fixed, thus firmly securing the first finger ring 100 and the second finger ring 200 to the transmission cord 310. In this way, according to the present invention, through using the transmission cord 310 as a transmission element in lieu of a motor transmission system, not only the mirror therapy device can be fabricated at significantly reduced cost, but it can also be ensured that the affected and healthy limbs move in synchronization, thus effectively avoiding asynchronous movements of the affected and healthy limbs due to inaccuracy and latency in signal detection. In order to ensure synchronous movements of the healthy and affected hands, after the first finger ring 100 and the second finger ring 200 are attached to the transmission cord 310, the transmission cord 310 is pre-tensioned so that the transmission cord 310 is always kept in a tensioned state during use.

As shown in Fig. 4, when the healthy finger performs a flexion movement, the first finger ring 100 is pushed down, causing the first cord segment 311 of the transmission cord 310 to move downward. As a result, the various cord segments of the transmission cord 310 are driven to move correspondingly. Wherein, the second cord segment 312 to which the second finger ring 200 is attached also moves downward, and the downward moving second cord segment 312 causes the second finger ring 200 and hence the affected finger inserted therein to make a synchronous flexion movement.

Finger rehabilitation for the patient is largely affected by friction and extension of the transmission cord 310. Therefore, the transmission cord 310 is made of a material with a relatively low coefficient of friction, and lubricating grease is applied to the surface of the transmission cord 310 during use, in order to achieve reduced friction. In order to reduce extension of the transmission cord 310 and disable its elastic deformation under stress, preferably, according to the present invention, the transmission cord 310 may be implemented as a Dyneema yarn, a Kevlar yarn or a steel cord with a diameter of 0.1-3 mm.

Preferably, as shown in Figs. 3 and 4, the first cord segment 311 to which the first finger ring 100 is attached is oriented at a first angle with respect to a horizontal plane, and the second cord segment 312 to which the second finger ring 200 is attached is oriented at a second angle with respect to the horizontal plane. Both the first and second angles are greater than 0° and less than 90°. This arrangement can additionally simplify the overall structure of the mirror therapy device provided in the present invention and readily enables, while the healthy limb is moving, the first finger ring 100 to drive the transmission cord 310 and hence the second finger ring 200 to move synchronously to make the affected limb repeat the same movements as the healthy limb, in particular finger flexion and extension movements, without interference between the movement trajectory with the transmission cord 310, thus achieving better rehabilitation outcomes.

Preferably, as shown in Figs. 2 to 4, the first finger ring 100 includes, connected to each other, a first ring body 110 and a first connecting member 120, and the second finger ring 200 includes, connected to each other, a second ring body 210 and a second connecting member 220. The transmission cord 310 includes a first transmission cord body 313 and a second transmission cord body 314. One end of the first transmission cord body 313 is connected to the first ring body 110, and the other end is connected to the second connecting member 220. One end of the second transmission cord body 314 is connected to the second ring body 210, and the other end is connected to the first connecting member 120. This arrangement can ensure that the first cord segment 311 to which the first finger ring 100 is attached is kept on the same straight line at every portion and that the second cord segment 312 to which the second finger ring 200 is attached is also kept on the same straight line at every portion, thus further ensuring that the first finger ring 100 and the second finger ring 200 can be maintained in synchronous movement and enabling the mirror therapy device provided in the present invention to achieve further improved therapeutic outcomes.

Preferably, as shown in Figs. 2 to 4, the transmission elements further include a plurality of first guide elements 320 and a plurality of second guide elements 330. The plurality of first guide elements 320 are arranged in correspondence with the first finger ring 100, and the plurality of second guide elements 330 are arranged in correspondence with the second finger ring 200. The transmission cord 310 is wound successively on the plurality of first guide elements 320 and the plurality of second guide elements 330 so as to form the aforesaid loop. The transmission cord 310 is able to reciprocate relative to the first guide element 320 and the second guide element 330. The first guide elements 320 and the second guide elements 330 can guide the transmission cord 310 so that the transmission cord 310 can respond at a faster rate during movement of the healthy finger, additionally ensuring synchrony of movement of the patient's hands.

Preferably, as shown in Figs. 2 to 4, at least two adjacent ones of the first guide elements 320 are arranged at different heights, with the first finger ring 100 being located between these two first guide elements 320, and at least two adjacent ones of the second guide elements 330 are arranged at different heights, with the second finger ring 200 being located between these two second guide elements 330. Arranging the first finger ring 100 between the two first guide elements 320 that are situated at different heights can keep the first cord segment 311 to which the first finger ring 100 is attached inclined from the horizontal plane, and arranging the second finger ring 200 between the two second guide elements 330 that are situated at different heights can keep the second cord segment 312 to which the second finger ring 200 is attached inclined from the horizontal plane. As such, when the healthy finger inserted in the first finger ring 100 performs a flexion or extension movement, the transmission cord 310 can be better driven to move correspondingly, thus ensuring synchrony of movement of the affected and healthy fingers.

Preferably, as shown in Figs. 2 to 4, in order to facilitate mounting of the first guide elements 320 and the second guide elements 330, the support mounts 340 are disposed inside the box body 700, the first guide elements 320 and the second guide elements 330 may be disposed inside the box body 700 by mounting the first guide elements 320 and the second guide elements 330 on support mounts 340 respectively.

Preferably, as shown in Figs. 2 to 4, the first guide elements 320 and the second guide elements 330 may all be pulleys. Implementing the guide elements for the transmission cord 310 as pulleys can not only effectively reduce friction of the transmission cord 310 and hence discomfort of the patient during limb training but also further ensure synchrony of movement of the affected and healthy limbs. In other embodiments, it is also possible that some of the first guide elements 320 and/or some of the second guide elements 330 are pulleys while the remaining first guide elements 320 and/or the remaining second guide elements 330 are not pulleys.

Preferably, as shown in Figs. 2 to 4, the mirror therapy device further includes at least one displacement sensor 400 arranged in correspondence with the transmission cord 310 and adapted to measure movement and displacement of the transmission cord 310. Arranging the displacement sensor 400 enables movement and displacement measurement of the transmission cord 310 using the displacement sensor 400. Since the transmission cord 310 does not elastically deform and is pre-tensioned at the very beginning, any amount of displacement of the transmission cord 310 represents the same amount of displacement resulting from finger flexion. Therefore, displacement resulting from finger flexion can be obtained by measuring displacement of the transmission cord 310. Thus, the displacement sensor 400 can measure finger movements in real time, providing a basis for interesting rehabilitation.

Preferably, the displacement sensor 400 is a photoelectric displacement sensor, and the transmission cord 310 is provided thereon with a mark corresponding to the displacement sensor 400. Compared with conventional inertial sensors, the photoelectric displacement sensor provides faster response and higher measurement accuracy, less suffers from zero shifting and is more reliable. It can measure finger movements in real time, providing a basis for interesting rehabilitation. Additionally, arranging the mark corresponding to the photoelectric displacement sensor on the transmission cord 310 can facilitate movement and displacement measurement of the transmission cord 310 by the photoelectric displacement sensor. In an initial configuration of the transmission cord 310, the mark is exactly aligned with the photoelectric displacement sensor, corresponding to a displacement measurement of zero. When the transmission cord 310 moves, the mark will move with the transmission cord 310, and an amount of displacement of the mark represents an amount of displacement of the transmission cord 310. Specifically, the mark may be a marking dot provided on the transmission cord 310, or a guide sheet provided on the transmission cord 310. As would be appreciated by those skilled in the art, the mark may also be another type of mark provided on the transmission cord 310, as long as it can provide a marking effect. The present invention is not limited to any particular type of mark.

Preferably, as shown in Figs. 2 to 4, in order to further improve measurement accuracy of the displacement sensor 400, the displacement sensor 400 is disposed between a first guide element 320 and a second guide element 330 which is provided in correspondence with and at the same height as the first guide element 320.

According to the above concept, the present invention also provides a mirror therapy system including a display device and the mirror therapy device as described above. The display device is adapted to display images of movements of the healthy and affected limbs. Since the mirror therapy system provided in the present invention includes the mirror therapy device as described above, it enables the healthy limb to drive the affected limb to repeat the same movements as the healthy limb to establish neural connections for synchronous vision, motor perception and proprioception of the patient's healthy and affected sides and better stimulate nerve recovery of the patient, thus addressing the problem of availability of only visual stimulation and absence of motor-perceptive and proprioceptive stimulation associated with conventional mirror therapy approaches. Moreover, since the mirror therapy system provided in the present invention also includes the display device, it can display images of movements of the healthy and affected limbs on the display device to allow the patient to clearly view the limb movements. This enables mirror therapy to provide an increased sense of reality and thus promote recovery of the patient's motor perception and proprioception. Specifically, the display device may be a computer monitor capable of displaying images captured by the aforementioned at least one first camera 610 and at least one second camera 620 in real time. In order to further promote recovery of the patient's motor perception and proprioception, the display device may apply an image compensation algorithm, preferably a neural network-based image compensation algorithm, to the display of the images of movements of the healthy and affected limbs to eliminate the influence of the transmission elements on limb movements by masking the transmission elements so that the patient will only see movements of the limbs, will not know the transmission mechanism and will imagine his/her nerve recovery. This enables mirror therapy to provide an additionally increased sense of reality and achieve better therapeutic outcomes. In other embodiments, the display device may also be adapted to display only images of movements of the affected limb to achieve mirror therapy rehabilitation.

To sum up, the mirror therapy device and system provided in the present invention has the following advantages over the prior art:
(1) The mirror therapy device provided in the present invention includes a first positioning assembly, a second positioning assembly and a transmission assembly connecting the first and second positioning assemblies, the first and second positioning assemblies arranged in opposition to each other in such a manner that both are able to reciprocate, the first positioning assembly adapted for positioning of a healthy limb of a patient, the second positioning assembly adapted for positioning of an affected limb of the patient, the first and second positioning assemblies relying on the transmission assembly for power transmission therebetween so that the second positioning assembly is able to move in synchronization with the first positioning assembly under the action of the transmission assembly. Therefore, with the mirror therapy device provided in the present invention, the healthy limb can drive the affected limb under the action of the transmission assembly to enable the affected limb to synchronously repeat the same movements as the healthy limb to establish neural connections for synchronous vision, motor perception and proprioception of the patient's healthy and affected sides and better stimulate nerve recovery of the patient, thus addressing the problem of availability of only visual stimulation and absence of motor-perceptive and proprioceptive stimulation associated with conventional mirror therapy approaches.
(2) The mirror therapy device provided in the present invention employs a transmission cord as a transmission element in lieu of a motor transmission system. As a result, not only the mirror therapy device can be fabricated at significantly reduced cost, but it can also be ensured that the affected and healthy limbs move in synchronization, thus effectively avoiding asynchronous movements of the affected and healthy limbs due to inaccuracy and latency in signal detection.
(3) The mirror therapy device provided in the present invention employs pulleys as guide elements for the transmission cord. This can not only effectively reduce friction of the transmission cord and hence discomfort of the patient during limb training but also further ensure synchrony of movement of the affected and healthy limbs.
(4) The mirror therapy device provided in the present invention employs a photoelectric displacement sensor for movement and displacement measurement of the transmission cord. Compared with conventional inertial sensors, the photoelectric displacement sensor provides faster response and higher measurement accuracy, less suffers from zero shifting and is more reliable. It can measure limb movements in real time, providing a basis for interesting rehabilitation.
(5) The mirror therapy device provided in the present invention employs a first camera capable of capturing movements of the healthy limb in real time and a second camera capable of capturing movements of the affected limb in real time. Moreover, it employs an image compensation algorithm for processing the captured images to remove the first positioning assembly, the second positioning assembly and the transmission assembly therefrom, thus avoiding the patient from knowing the transmission mechanism and enabling mirror therapy to provide an increased "sense of reality". Further, tactile differences perceived by the patient during transmission can be compensated for by adding immersion from interesting training, in order to further enhance the patient's nerve recovery.
(6) Since the mirror therapy system provided in the present invention includes the mirror therapy device as described above, it has all the advantages of the mirror therapy device. The mirror therapy system provided by the present invention enables the healthy limb to drive the affected limb to allow the affected limb to repeat the same movements as the healthy limb, thus establishing neural connections for the patient's vision, motor perception and proprioception and better stimulating nerve recovery of the patient.

The foregoing description is merely that of preferred embodiments of the present invention and is not intended to limit the scope of the invention in any sense. Any and all changes and modifications made by those of ordinary skill in the art to which the present invention pertains based on the above disclosure all fall within the scope of the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A mirror therapy device, comprising a first positioning assembly, a second positioning assembly and a transmission assembly connecting the first and second positioning assemblies,
the first and second positioning assemblies arranged in opposition to each other in such a manner that both are able to reciprocate, the first positioning assembly configured for positioning of a healthy limb of a patient, the second positioning assembly configured for positioning of an affected limb of the patient,
the transmission assembly configured for power transmission between the first and second positioning assemblies so that the second positioning assembly moves in synchronization with the first positioning assembly.

2. The mirror therapy device according to claim 1, wherein the first positioning assembly comprises at least one first finger ring and the second positioning assembly comprises at least one second finger ring each arranged in correspondence with a respective one of the at least one first finger ring, the transmission assembly comprising at least one set of transmission elements each configured to maintain a respective one of the at least one first finger ring and a respective one of the at least one second finger ring that is arranged in correspondence with the respective finger ring in synchronous movement.

3. The mirror therapy device according to claim 2, wherein the transmission elements include a transmission cord capable of reciprocating and tied together at both ends to form a loop, wherein the first finger ring is attached to a first cord segment of the transmission cord and the second finger ring is attached to a second cord segment of the transmission cord, and wherein the first cord segment to which the first finger ring is attached is made in the same movement state as the second cord segment to which the second finger ring is attached.

4. The mirror therapy device according to claim 3, wherein the first cord segment to which the first finger ring is oriented at a first angle with respect to a horizontal plane and the second cord segment to which the second finger ring is oriented at a second angle with respect to the horizontal plane, both the first and second angles being greater than 0° and less than 90°.

5. The mirror therapy device according to claim 3, wherein the first finger ring comprises, connected to each other, a first ring body and a first connecting member and the second finger ring comprises, connected to each other, a second ring body and a second connecting member, the transmission cord comprising a first transmission cord body and a second transmission cord body, the first transmission cord body connected to the first ring body at one end and to the second connecting member at the other end, the second transmission cord body connected to the second ring body at one end and to the first connecting member at the other end.

6. The mirror therapy device according to claim 3, wherein the transmission element further include a plurality of first guide elements and a plurality of second guide elements, the plurality of first guide elements arranged in correspondence with the first finger ring, the plurality of second guide elements arranged in correspondence with the second finger ring, wherein the transmission cord is successively wound on the plurality of first guide elements and the plurality of second guide elements so as to form the loop and be able to reciprocate relative to the first and second guide elements.

7. The mirror therapy device according to claim 6, wherein the first and second guide elements are all pulleys.

8. The mirror therapy device according to claim 3, further comprising at least one displacement sensor arranged in correspondence with the transmission cord, and the displacement sensor being configured to measure displacement of the transmission cord.

9. The mirror therapy device according to claim 8, wherein the displacement sensor is a photoelectric displacement sensor, and wherein the transmission cord is provided thereon with a mark corresponding to the displacement sensor.

10. The mirror therapy device according to claim 1, further comprising a first support pad configured for supporting the healthy limb and a second support pad configured for supporting the affected limb, the first support pad arranged in correspondence with the first positioning assembly, the second support pad arranged in correspondence with the second positioning assembly.

11. The mirror therapy device according to claim 10, wherein the first support pad is provided with a first concave surface on its side intended to be brought into contact with the healthy limb, and wherein the second support pad is provided with a second concave surface on its side intended to be brought into contact with the affected limb.

12. The mirror therapy device according to claim 1, further comprising at least one first camera and at least one second camera, the first camera arranged in correspondence with the first positioning assembly, the second camera arranged in correspondence with the second positioning assembly.

13. The mirror therapy device according to claim 12, further comprising a lighting lamp.

14. The mirror therapy device according to claim 1, further comprising a box body defining an internal cavity where the first positioning assembly, the second positioning assembly and the transmission assembly are all disposed.

15. The mirror therapy device according to claim 14, wherein the box body comprises a support frame and, all mounted on the support frame, a first side plate, a second side plate, a top plate, a back panel and a bottom plate.

16. The mirror therapy device according to claim 15, wherein a gap is left between the bottom plate and the bottom of the support frame so that the bottom plate is raised above the bottom of the support frame.

17. The mirror therapy device according to claim 15, wherein the box body further comprises a front panel mounted on the support frame, the front panel arranged in opposition to the back panel, the front panel provided therein with a first aperture configured for passage of the healthy limb therethrough and a second aperture configured for passage of the affected limb therethrough, the first aperture arranged in correspondence with the first positioning assembly, the second aperture arranged in correspondence with the second positioning assembly.

18. A mirror therapy system, comprising the mirror therapy device of any one of claims 1 to 17 and a display device, the display device configured to display images of movements of the healthy and affected limbs.
